# EUROPEAN PATENT APPLICATION

(11) **EP 4 566 578 A1**
(43) Date of publication of application: **11.06.2025**
(21) Application number: 23213894.1
(22) Date of filing: 04.12.2023
(51) Int. Cl.: A61F 13/495, A61F 5/443, A61F 5/451

(54) **DISPOSABLE DEFECATION POCKET**

(71) Applicant: The Procter & Gamble Company, Cincinnati, OH 45202 (US)
(72) Inventor: SURUSHE, Abhishek, 65824 Schwalbach am Taunus (DE)
(74) Representative: P&G Patent Germany

(57) **Abstract**

A disposable defecation pocket for containing feces is disclosed. The pocket has an opening, a perimeter around the opening, a bottom, and a side wall. The pocket is able to expand from a flat, collapsed configuration to an erected configuration, wherein the erected configuration provides a void volume inside the pocket. The pocket is, at least partly, in its erected configuration in the absence of any applied pressure.

## Description

### FIELD OF THE INVENTION

The present invention relates to a disposable defecation pocket for containing feces. The defecation pocket is a faecal management device for babies, children or adults. The defecation pocket is to be attached directly to the wearer's skin between the buttocks of the wearer. The defecation pocket is also to be attached to an article, such as an absorbent article or underwear. In use, the defecation pocket is positioned between wearer's skin and the article. Attachment to the skin and the article ensures maintenance of the device in the desired position. The defecation pocket is able to convert from a flat configuration to an erected configuration on its own motion.

### BACKGROUND OF THE INVENTION

Fecal management devices are known articles of manufacture that are designed to be worn principally by babies, toddlers but also by adults, in particular by bedridden patients. Such devices are attached to the anal region and are intended to receive entrap and immediately contain faecal material. Known fecal management devices are separately used on their own. Other known fecal management devices are comprised by or attachable to absorbent articles, such as diapers.

Often, known fecal management devices are folded and flat when empty. Such devices are typically expanded passively upon exposure of feces inside the device. Hence, the fecal material exerts pressure on the device, leading to expansion of the device. This can lead to an awkward feeling of pressure upon defecation or even hinder proper defecation. Also, the storage capacity of the device can be detrimentally affected. Improper expansion of the pocket leading to insufficient void volume (hence, storage capacity) can also cause leakage of feces as the pressure exerted by the feces on the attachment between device and wearer's skin can result in unintentional partial or complete detachment from the wearer's skin. Such an occurrence is unacceptable causing distressing consequences for both the wearer and the carer.

Another disadvantage often associated with known fecal management devices is that they may not stick in their proper position, e.g. upon motions of the wearer, leading to a discomfort of the wearer due to an uncomfortable position of the device, or even to detachment of the device from the wearer's skin.

There is thus a need for a defecation management device that securely receives and captures feces, creates as little discomfort of the wearer as possible, and reduces the risk of feces not entering or leaking out of the device due to improper inflation of the device or detachment from the wearer's skin.

### SUMMARY OF THE INVENTION

The invention relates to a disposable defecation pocket (hereinafter referred to as 'pocket') for containing feces. The pocket has an opening, a perimeter around the opening, a bottom, and a side wall extending from the perimeter to the bottom. The side wall and the bottom each have a first surface facing inwardly (i.e. towards the inside of the pocket) and a second surface facing outwardly).

The pocket is able to expand from a flat, collapsed configuration to an erected configuration, wherein the erected configuration provides a void volume inside the pocket. The pocket is, at least partly, in its erected configuration in the absence of any applied pressure. `Applied pressure' means any externally applied pressure other than gravity.
The pocket comprises
- a first material forming the bottom,
- a second material forming the side wall,
   wherein the first and second material is the same or is different from one another;
- a first attachment material, such as a body adhesive, continuously provided along the perimeter, preferably being provided continuously along the perimeter, wherein the first attachment material enables attachment of the perimeter to the skin around the anus of a wearer;
- a second attachment material provided on the second surface of the bottom, the second attachment material, such as an adhesive or a mechanical attachment material, enabling attachment of the bottom to a surface of an article selected from the group consisting of a diaper, an absorbent pant, an absorbent insert, an outer cover, and an underpants.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a top view of an example pocket;
Fig. 2 is a bottom view of an example pocket with a portion of the bottom being peeled away for illustrative purposes;

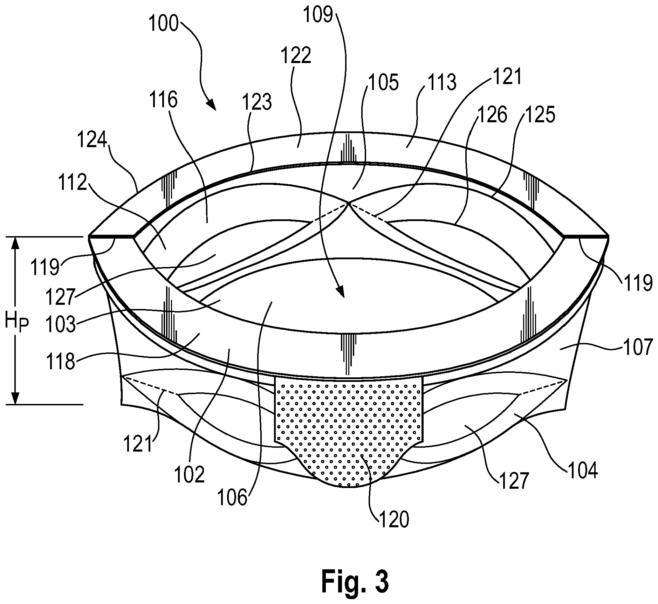

Fig. 3 is a side view of an example pocket in its erected configuration;
Fig. 4 is a side view of an example pocket in a substantially flat configuration;
Fig. 5 shows an example side wall that is intended to be comprised by a pocket of the present invention;

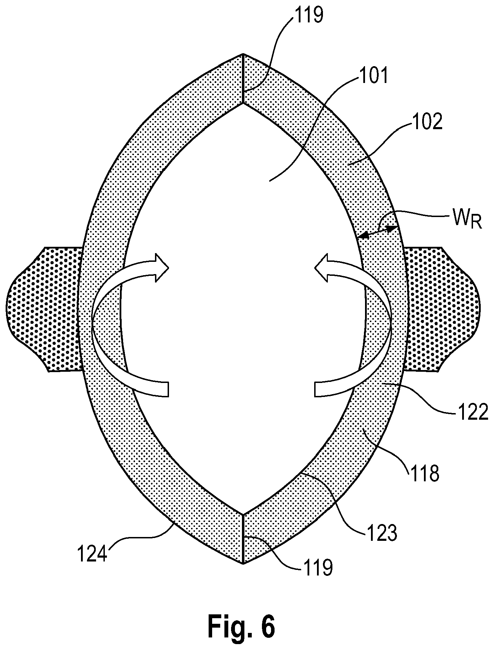

Fig. 6 is a top view of an example pocket with an arrow indicating the way in which the pocket can be closed for disposal after use;

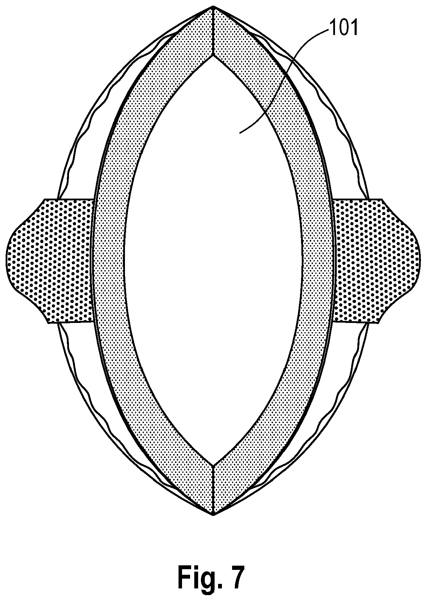

Fig. 7 is a top view of the pocket of Fig. 6 in a partially closed configuration;

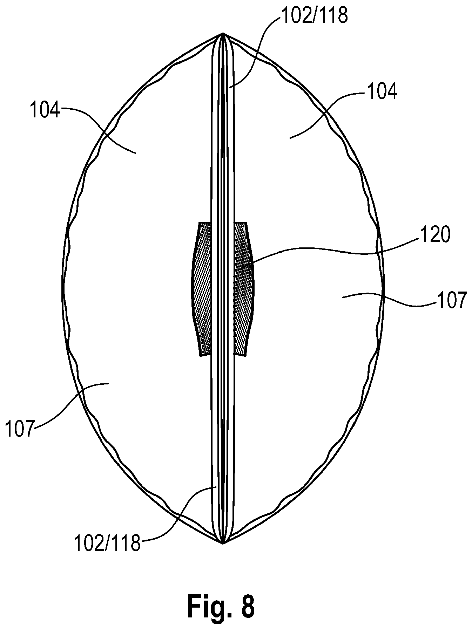

Fig. 8 is a top view of the pocket of Fig. 6 in its closed configuration for disposal;

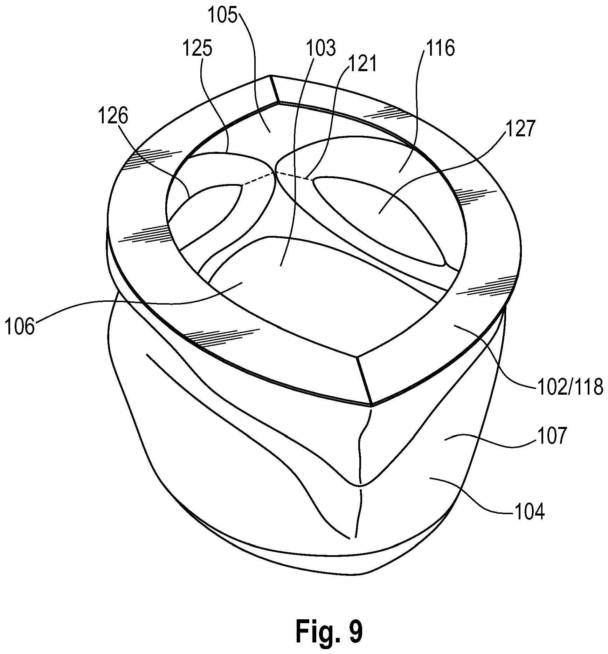

Fig. 9 is another side view of an example pocket in its erected configuration;
Fig. 10 is a plan view of an example article in the form of a taped diaper, garment-facing surface facing the viewer, in a flat laid-out state;
Fig. 11 is a plan view of the example article of Fig. 10, wearer-facing surface facing the viewer, in a flat laid-out state;
Fig. 12 is a front perspective view of the article of Figs. 10 and 11 in a fastened position;
Fig. 13 is a front perspective view of an article in the form of an absorbent pant;
Fig. 14 is a rear perspective view of the article of Fig. 13;
Fig. 15 is a plan view of the article of Fig. 13, laid flat, with a garment-facing surface facing the viewer;
Fig. 16 is a cross-sectional view of the article taken about line 16-16 of Fig. 15;
Fig. 17 is a cross-sectional view of the article taken about line 17-17 of Fig. 15;
Fig. 18 is a plan view of an example absorbent core or an article;
Fig. 19 is a cross-sectional view, taken about line 19-19, of the absorbent core of Fig. 18;
Fig. 20 is a cross-sectional view, taken about line 20-20, of the absorbent core of Fig. 18;

### DETAILED DESCRIPTION OF THE INVENTION

### Definitions

As used herein, the following terms shall have the meaning specified thereafter:
"Absorbent article" refers to devices that absorb and contain body exudates, particularly urine and other water-containing liquids, and, more specifically, refers to devices that are placed against or in proximity to the body of the wearer to absorb and contain the various exudates discharged from the body. Absorbent articles may include diapers (for babies and infants as well as for adult incontinence), pants (for babies and infants as well as for adult incontinence). As used herein, the term "exudates" includes, but is not limited to, urine, blood, vaginal discharges, sweat and fecal matter. Preferred absorbent articles of the present invention are disposable absorbent articles, more preferably disposable diapers, disposable absorbent pants and disposable absorbent inserts.

"Absorbent core" is used herein to refer to a structure intended to be disposed between a topsheet and backsheet of an absorbent article for absorbing and storing liquid received by the absorbent article.

"Disposable" is used in its ordinary sense to mean an article that is disposed or discarded after a limited number of usage events over varying lengths of time, for example, less than 10 events, less than 5 events, or less than 2 events. If the disposable article is a defecation pocket or is a diaper, an absorbent pant, or an absorbent insert, the disposable article is most often intended to be disposed after single use.

"Diaper" and "absorbent pant" refers to an absorbent article generally worn by babies, infants and incontinent persons about the lower torso so as to encircle the waist and legs of the wearer and that is specifically adapted to receive and contain urinary and fecal waste. In an absorbent pant, as used herein, the longitudinal edges of the first and second waist region are attached to each other to a pre-form waist opening and leg openings. An absorbent pant is placed in position on the wearer by inserting the wearer's legs into the leg openings and sliding the absorbent pant into position about the wearer's lower torso. An absorbent pant may be pre-formed by any suitable technique including, but not limited to, joining together portions of the absorbent pant using refastenable and/or non-refastenable bonds (e.g., seam, weld, adhesive, cohesive bond, fastener, etc.). An absorbent pant may be pre-formed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

"Belt-type pant" refers to an absorbent pant with a ring-like elastic belt comprising an absorbent main body to cover the crotch region of the wearer, a front elastic belt and a back elastic belt. The absorbent main body extends into and overlaps with the front and back elastic belt. The front and back elastic belts form a discrete elastic belt extending transversely defining the waist opening and being discontinuous in the longitudinal direction. For the belt-type pant, the discrete ring-like elastic belt may also be referred to as the elastic belt. For the belt-type pant the front and back elastic belts jointly define the waist opening and the front and back elastic belt together with the absorbent main body define the leg openings.

"Flat configuration" of the pocket, as used herein, refers to a configuration wherein the pocket is compressed in its height direction such that the materials comprised by the pocket are compressed to a substantially flat. The flat configuration may correspond to a pocket height of 1% to 10%, or from 1% to 8%, or from 1% to 5% of the maximum height of the pocket in its fully erected configuration. The compressed pocket height will mainly depend on the thickness of the materials comprised by the pocket and "piling up" on top of one another.

"Longitudinal" refers to a direction running substantially perpendicular from a waist edge to an opposing waist edge of the article and generally parallel to the maximum linear dimension of the article. "Transverse" refers to a direction perpendicular to the longitudinal direction.

"Film" refers to a sheet-like material wherein the length and width of the material far exceed the thickness of the material. Typically, films have a thickness of about 0.5 mm or less.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is equal to or greater than 90° and the fluid does not spread spontaneously across the surface of the fiber. The contact angle test method used for the present invention is set out herein below.

As used herein, the term "nonwoven web" refers to a material which is a manufactured web/layer of directionally or randomly oriented fibers or filaments. The fibers may be of natural or man-made origin. Natural fibers may be selected from the group consisting of wood pulp fibers, wheat straw fibers, rice straw fibers, flax fibers, bamboo fibers, cotton fibers, jute fibers, hemp fibers, sisal fibers, bagasse fibers, Hesper aloe fibers, miscanthus, marine or fresh water algae/seaweeds, silk fibers, wool fibers, and combinations thereof. Another group of fibers may also be regenerated cellulose fibers, such as viscose, Lyocell (Tencel^{®}), rayon, modal, cellulose acetate fibers, acrylic fibers, cuprammonium rayon, regenerated protein fibers etc. Preferably, the natural fibers or modified natural fibers are selected from the group consisting of cotton fibers, bamboo fibers, viscose fibers or mixtures thereof. Preferably, the natural fibers are cotton fibers. Synthetic fibers may be selected from the group consisting of polyolefins (such as polyethylene, polypropylene or combinations and mixtures thereof), polyethylene terephthalate (PET), co PET, polylactic acid (PLA), polybutylene succinate (PBS), polyhydroxy alkanoates (PHA), nylon (or polyammide), or mixtures or combinations thereof. An alternative option is to use superabsorbent fibers, for example SAF^{™} which is a cross-linked terpolymer based on acrylic acid, which is partially neutralised to its sodium salt, commercially available from Technical Absorbents.

The fibers in a nonwoven web are consolidated by friction, and/or cohesion and/or adhesion, and/or by heat bonding, pressure bonding, heat and pressure bonding, and/or ultrasonic bond excluding paper and products which are woven, knitted, tufted, stitch-bonded. The fibers may be staple fibers (e.g. in carded nonwoven webs) or continuous fibers (e.g. in spunbonded or meltblown nonwoven webs).

Nonwoven webs can be formed by many processes such as meltblowing, spunlaying, solvent spinning, electrospinning, and carding, and the fibers can be consolidated, e.g. by hydroentanglement (in spunlaced nonwoven webs), air-through bonding (using hot air that is blown through the fiber layer in the thickness direction), needle-punching, one or more patterns of bonds and bond impressions created through localized compression and/or application of heat or ultrasonic energy, or a combination thereof. The fibers may, alternatively or in addition, be consolidated by use of a binder. The binder may be provided in the form of binder fibers (which are subsequently molten) or may be provided in liquid, such as a styrene butadiene binder. A liquid binder is provided to the fibers (e.g. by spraying, printing or foam application) and is subsequently cured to solidify.

The basis weight of nonwoven webs is usually expressed in grams per square meter (g/m²).

In a spunlace nonwoven web the fibers have been carded as precursor web and then subjected to hydroentanglement to intermingle and intertwine the fibers with each other. Cohesion and the interlacing of the fibers with one another may be obtained by means of a plurality of jets of water under pressure passing through a moving fleece or cloth and, like needles, causing the fibers to intermingle with one another (hereinafter also referred to as "hydraulic interlacing"). Thus, consolidation of a spunlace nonwoven web is essentially a result of hydraulic interlacing.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" encompasses the narrower terms "consisting essential of" which excludes any element, step or ingredient not mentioned which materially affect the way the feature performs its function, and the term "consisting of' which excludes any element, step, or ingredient not specified.

"Adjacent" and "adjacent to", as used herein, means very near or in close proximity. "adjacent" may mean spaced by a distance of no more than 50 mm, or not more than 40 mm, or not more than 30 mm, or not more than 25 mm, or not more than 20 mm, or not more than 15 mm, or not more than 10 mm, or not more than 5 mm. For example, if the absorbent article is a relatively large absorbent article intended for adult incontinent wearers, "adjacent" may mean spaced by a distance of no more than 50 mm. In another example, where the absorbent article is intended to be worn by babies, "adjacent" may mean spaced by a distance of not more than 25 mm, or not more than 20 mm, or not more than 15 mm, or not more than 10 mm, or not more than 5 mm.

### Disposable defecation pocket

The invention relates to a disposable defecation pocket 100 for containing feces. The pocket can be used with a disposable absorbent article, such as a diaper, a pant or an absorbent insert. Alternatively, the pocket 100 can be used with an outer cover (which is typically used in combination with a disposable absorbent insert) or with reusable underpants. The absorbent article may be disposable or reusable. The outer cover may be reusable or disposable.

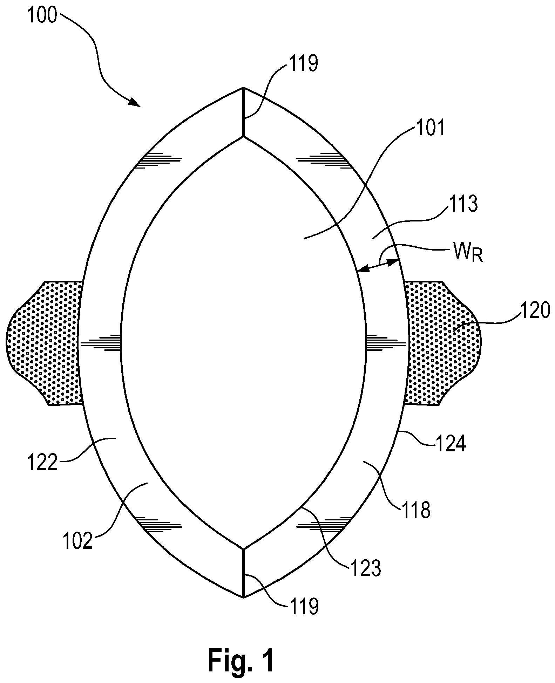

As is exemplarily shown in Fig. 1, the pocket 100 comprises an opening 101 and a perimeter 102 around the opening. The perimeter 102 is configured to be applied to the wearer's skin around the anus.

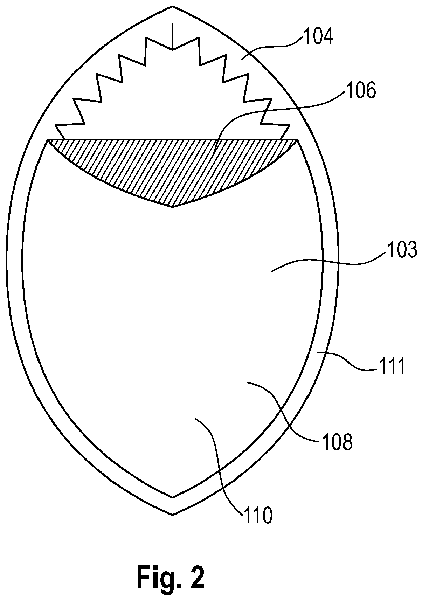

As is exemplarily shown in Figs. 2 an 3, the pocket 100 further comprises a bottom 103 and a side wall 104 extending from the perimeter 102 to the bottom 103. The side wall 104 and the bottom 103 each have a first surface 105, 106 facing inwardly and a second surface 107, 108, opposite the first surface, which is facing outwardly.

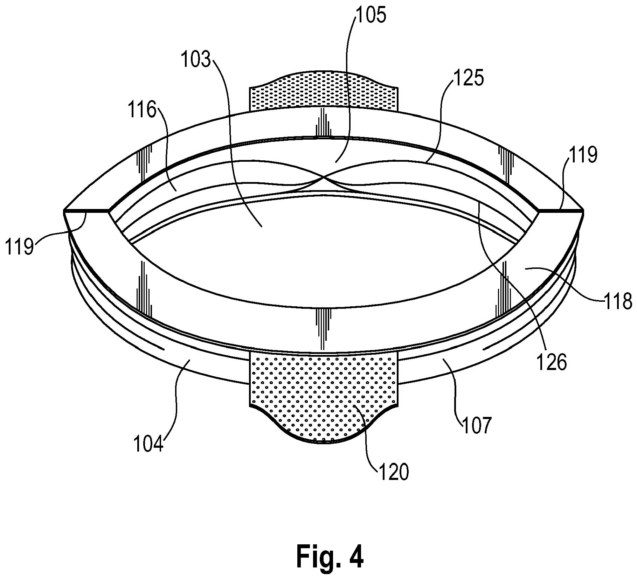

The pocket 100 is able to expand from a flat, collapsed configuration to an erected configuration, wherein the erected configuration provides a void volume 109 inside the pocket. The void volume 109 is intended to capture feces when the pocket is applied around the anus of a wearer. A pocket in its substantially flat configuration is exemplarily shown in Fig. 4.

The appropriate volume of the pocket (i.e. the void volume) depends on the intended use of the pocket, i.e. whether it is intended to be used in an article worn by babies, toddlers or adults. The volume of the pocket may be at least 100,000 mm³, or at least 100,000 mm³, or at least 150,000 mm³. The volume of the pocket may not be more than 400,000 mm², or not more than 350,000 mm³, or not more than 300,000 mm³.

In the absence of any applied pressure, the pocket 100 is, partly or completely, in its erected configuration. When the pocket 100 is completely erected, the void volume 109 inside the pocket 100 has the maximum volume, whereas the void volume 109 in the flat, collapsed configuration is very small, it may be less than 50,000 mm³, or less than 30,000 mm³, or less than 20,000 mm², or less than 10,000 mm³, or it may even be zero.

The fact that the pocket is at least partly in its erected configuration in the absence of any applied pressure means does not only mean that the pocket remains in an -at least partly- erected configuration, but it also means that the flat compressed pocket will assume an -at least partly-erected configuration on its own motion if no external pressure is applied. If the pocket is comprised in a package under high compression, the pocket may not readily erect on its own motion immediately after it has been taken out of the package. In these circumstances, it may take a while until the pocket erects. For the present invention, the time it takes for the pocket to at least partly erect is not more than 60 minutes after it has been taken out of a package.

"Absence of any applied pressure", as used herein, means that no pressure is exerted on the pocket other than gravity. A pressure may, e.g., be applied when one or more pockets are comprised in a package for sale or transport. Another example of an applied pressure is the pressure that a wearer's body exerts on the pocket, when the pocket is installed onto an absorbent article, underwear or outer cover and the article is in use. Also, the clothes of a wearer can apply pressure on the pocket when the pocket is used.

By having a pocket that is at least partly in its erected configuration in the absence of any applied pressure, defecation can be efficiently disburdened as pocket does not need to be opened by the feces entering the pocket.

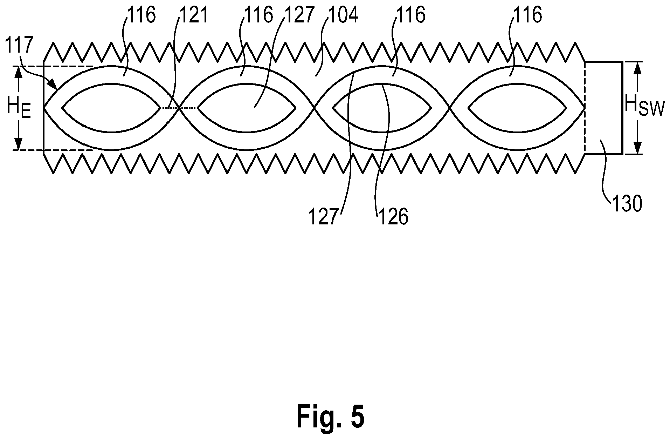

The pocket 100 comprises a first material 110 forming the bottom 103 and a second material 111 forming the side wall 104. The first and second material 110, 111 may be the same or different. If the first and second material 110, 111 are the same, the bottom 103 and side wall 104 may be formed of one continuous material. Alternatively, the bottom 103 and side wall 104 may be formed separately (also when they are made of the same material) and attached to each other subsequently, e.g. by use of an adhesive. One way of attaching the side wall 104 to the bottom 103 is by providing either the bottom, the side wall or both, with extensions around the edges, which then serve as an attachment area which is adhesively attached to the bottom or side wall, respectively. Such an attachment area is exemplarily shown as zig-zag formed extensions of the side wall in Figs. 2 and 5.

The perimeter 102 of the pocket 100 may form a continuous rim 118 around the opening. The rim 118 may be formed continuously with the side wall 104 or may be formed separately and subsequently attached to the side wall 104. The rim 118 has a first surface 122 facing away from the pocket 100. The first surface 122 will face towards the skin of the wearer when the pocket 100 is applied on a wearer. This first surface 122 of the rim 118 is intended to be attached onto the skin of a wearer around the anus. To facilitate attachment of the perimeter 102 to the skin, a first attachment material (not shown) is continuously provided along the perimeter 102, e.g., on the first surface 122 of the rim 118.

The rim 118 will have an inner and an outer perimeter 123, 124, the inner perimeter 123 defining the size and shape of the opening 101. The -shortest- distance between the outer and inner perimeter 123, 124 defines the width W_{R} of the rim 118. The rim 118 may have a width W_{R} of from 5 mmto 25 mm, or from 7mm to 20 mm, or from 10 mm to 20 mm.

The rim may be formed of any suitable material. For example, the rim may comprise or may be formed of a nonwoven web having a basis weight of at least 15 g/m², or at least 20 g/m², or at least 25 g/m², and not more than 120 g/m², or not more than 100 g/m², or not more than 80 g/m². The nonwoven web may be a spunbonded nonwoven web or a carded nonwoven web. The nonwoven web may comprise or may be formed of synthetic fibers. Suitable synthetic materials may be polyester, polypropylene, PET or combinations thereof. The fibers may be monocomponent fibers, bicomponent fibers (such as polyethylene / polypropylene bicomponent fibers) or mixtures thereof.

The size and shape of the opening 101 may be such that it enables attachment of the pocket 100 around the anus of a wearer, including the area between the anus and the genitals of a wearer. Thereby, the pocket 100 covers the anus but does not cover the genitals of the wearer. Thus, the pocket is able to collect feces but does not receive the wearer's urine, isolating feces away from the urine.

The size of the pocket 100 depends on the size dimensions of the wearer in the area around the anus. Thus, a larger pocket may be required for adult wearers vs. pockets for smaller children or even babies. The size and void volume 109 of the pocket will generally be adjusted not only to the size and dimensions of the intended wearer but also to the amount of fecal material that will typically be expected to be handled and captured by the pocket.

The shape of the pocket 100 is not especially limited as long as the perimeter is able to properly cover the area around the anus - and exclude the genitals of the wearer. The shape of the pocket's side wall 104 may be ellipsoid or circular. This means, that the side wall 104 of the pocket 100 in its erected configuration, when looking from above the opening, takes an ellipsoid or circular shape.

The first attachment material may be a body adhesive. A body adhesive should generally have a skin compatible composition and not be harsh or aggressive towards the skin or cause skin irritation or inflammation. The word "skin" according to the present invention does not only relate to the specific derma of the user but includes the mucous tissue as well as the hair which is typically found in the genital region. Moreover, it is also desirable to provide an adhesive such that the disposal human waste management device can be readily removed from the wearer, without the wearer experiencing any unacceptable pain level. This is particularly important under circumstances, where the device is misplaced, and removal and reapplication of the device once or even a number of times is required and or to ensure the application of such devices on sensitive skin and wearer groups such as infants. However, on the other hand the desired level of adhesion in dry and wet conditions, albeit painless should of course also be maintained during such multiple applications of the device.

Suitable adhesives for attachment onto the skin are described e.g. WO 98/03208 which discloses medical pressure sensitive adhesives which can adhere to dry or wet skin and which comprise a mixture of hydrophilic (meth)acrylate copolymer containing tertiaryamino groups, a hydrophilic (meth)acrylate copolymer containing carboxyl groups, carboxylic acids and a crosslinking system. Other adhesives for attachment onto the skin are described in US6878756 and EP0888763. Generally, the body adhesive desirably have- relatively low peel strength and relatively high shear strength. The body adhesives may be applied to the pocket with a high caliper (i.e. a body adhesive being applied with a thickness of 3 mm to 7 mm) to facilitate relatively low peel strength and relatively high shear strength.

However, as the pocket 100 is not only attached to the wearer's skin during use but is also intended to be attached to an article, the requirements on the adhesive strength of the body adhesive are considered to be less demanding versus a fecal management device which is only attached to the skin.

Alternatively, or in addition to a body adhesive, the first attachment material may also be silicone gel, such as silicone gel strips, e.g. available as Epi-Derm Silicone Gel Long Strips 5 paris by Biodermis | Amred-Amred Healthcare Ltd. Other materials suitable as first attachment material are nonwovens having micro vacuum cups embedded therein.

The first attachment material may be provided as a double-sided tape, wherein one side is attached on the perimeter of the pocket (e.g. on the rim of the pocket) and the other side comprises the first attachment material.

The first attachment material may be covered with a release liner (not shown). The release liner can help avoiding a premature attachment of the pocket onto a surface prior to attachment to the proper position of the wearer's skin.

A second attachment material (not shown) is provided on the second surface 108 of the pocket's bottom 103. The second attachment material is configured to enable attachment of the pocket's bottom 108 to a surface of an article which is selected from the group consisting of a diaper, an absorbent pant, an absorbent insert, an outer cover, and underpants. The second attachment material may attach the bottom 103 of the pocket 100 onto the surface of the article which, in the absence of the pocket, would otherwise be in direct contact with the area of and around the anus of the wearer. For a diaper, an absorbent pant or an absorbent insert, this may be the topsheet of the article.

The second attachment material may be an adhesive, such as an adhesive that is typically used to attach a sanitary napkin or a panty liner onto the wearer's underwear. The adhesive may be a pressure sensitive adhesive. Suitable adhesives are Fuller 2238 XZP,1377 XZP, HL-1258 or H-2031, all manufactured by H. B. Fuller Company of St. Paul, Minnesota. Other adhesives suitable as second attachment material are Century A-305-IV manufactured by the Century International Adhesives and Coatings Corporation of Columbus, Ohio; Instant LOK 34-2823 manufactured by the National Starch and Chemical Company of Bridgewater, New Jersey.

Alternatively, a mechanical fastener component may be used as second attachment material. For example, one or more patches of hook fasteners may form the second attachment material or may be comprised by the second attachment material. Such hook fasteners are well known as fastener components of hook and loop fasteners to attach a diaper onto a wearer around the waist. Patches of hook fasteners will work especially well if the pocket is used in a diaper, absorbent pant or absorbent insert, or reusable outer cover, as these articles may have nonwoven material as topsheet. Such nonwoven material can more readily engage with the hooks of a hook fastener compared to regular underpants, which are typically made of knitted fabric of, e.g. cotton, wool or silk. Also, hooks may deteriorate a woven or knitted fabric of regular underpants, such that an adhesive may be more suitable as second attachment material, when the pocket is predominantly intended for use with regular underpants.

The second attachment material may be covered by a release layer (not shown), which protects the second attachment material from premature attachment before the pocket 100 is to be attached to the (absorbent) article. Such release layers are well known in the art, e.g., for protecting adhesives of diapers, and may be formed of film material. The release layer may larger than the second attachment material.

The second attachment material may be applied continuously or discontinuously and may be provided on a part of, or the complete bottom 103 of the pocket 100. The bottom 103 of the pocket 100 may be flat when the pocket is in its fully erected configuration. In the fully erected configuration, the bottom 103 may either be completely flat or may be flat at least in the area of the second attachment material. This facilitates easy and proper attachment of the pocket to the intended area of the article.

If the article is a disposable article and is a diaper, absorbent pant, absorbent insert or outer cover, the second attachment material may be chosen such that the pocket 100 can be detached from the article after use without destroying or substantially deteriorating the surface of the article, to which the pocket 100 was attached. For example, when the bottom 103 of the pocket 100 is intended to be attached to a nonwoven topsheet of a disposable article, the second attachment material may be an adhesive that does not pull out a substantial number of fibers from the nonwoven topsheet, or even tear a hole into the topsheet. The same applies when the second attachment material is a hook fastener.

The article to which the pocket 100 can be attached, may have a longitudinal dimension and a transverse dimension orthogonal to the longitudinal dimension. The pocket may have a length which is intended to be aligned with the longitudinal dimension of the article, and a width which is configured to be aligned with the transverse dimension of the article. For the avoidance of doubt, while the length and width of the pocket may be aligned with the longitudinal and transverse dimension of the article, this does not mean that the pocket is intended to have the same length and width as the article. The length of the pocket may be considerably shorter than the longitudinal dimension of the article. The width of the pocket may be the same or smaller than the transverse dimension of the article. The pocket may be provided with suitable indicia (e.g. symbols, graphics or text printed on the pocket) to indicate proper alignment of the pocket's length and width with the article.

The pocket has its minimum height -extending from the bottom to the perimeter- in its flat, collapsed configuration and a maximum height in its fully erected configuration. The pocket may erect to at least 30%, or at least 50%, or at least 70% of its maximum height in the absence of any applied forces.

To configure the pocket 100 such that it is at least partly in its erected configuration the side wall 104 of the pocket may comprise a hinge 121. The hinge 121 can provide a fold line along which the pocket 100 folds when being compressed from its erected configuration to its flat, collapsed configuration. Compression occurs substantially parallel to the direction of the height H_{P} of the pocket. The folding may occur such that the side wall 104 folds inwardly towards the interior of the pocket, or folding occurs such that the side wall 10 fold outwardly, or folding occurs partly towards the interior of the pocket and partly outwardly.

The side wall 104 may comprise a third material 112. The third material 112 may be provided discontinuously. The third material 112 may be attached to the second material 111. In such configurations, the combination of the second and third material 111, 112 enables the at least partial - or complete- erection of the pocket 100 in the absence of any applied pressure.

The first and second material 110, 111 may be nonwoven, film layers or combinations thereof. The nonwoven may be a spunbond nonwoven. The nonwoven may be made of polyolefin fibers, polypropylene fibers, or polyethylene terephthalate fibers. The film may be made of polyethylene or co-polymers thereof. The basis weight of the first and second material may be from 8 g/m² to 40 g/m², or from 8 g/m² to 30 g/m², or from 10 g/m² to 25 g/m² The second material may have a sufficient level of stiffness to help maintain the side wall (and thereby the pocket as a whole) in its erected configuration.

The second material may be provided as a rectangular material (exemplarily shown in Fig. 5) which is subsequently turned into a continuous side wall that is attached to the bottom. The rectangular material may comprise an attachment area 130, which is used to "close" the second material to form the side wall by attaching the attachment area to the respective other side edge of the rectangular material. The third material may not be provided in the attachment area 130 to avoid overlap of the third material in the side wall.

The materials forming the side wall 104 and bottom 103 should generally be able to securely store any fecal material deposited inside the pocket 100 to avoid any leakage or bleed through (especially for runny fecal material). The side wall 104 may have a low surface tension strikethrough time of at least 30 seconds according to the test method set out herein below. The bottom 103 may have a low surface tension strikethrough time of at least 100 secs according to the test method set out herein below.

The third material 112 may be a foam, such as an EPE (expanded polyethylene foam). Such foams are low density, semi-rigid, closed cell foams.

The third material 112 may be a carded material. The third material 112 may be formed of multiple layers of carded material. The carded material may be made of PE/PET fibers.

The third material 112 may have a basis weight of from 15 g/m² to 100 g/m², or from 30 g/m² to 100 g/m², or from 30 g/m² to 70 g/m².

The third material 112 may be provided as several elliptical, oval, eye shaped, or circular elements 116, each element 116 having an opening 127 therein. In one embodiment, the third material is provided as several eye-shaped elements.

The elements 116 may be applied as a row 117 extending along the side wall 104. The height H_{E} of each element 116 may be from 60% to 100%, or from 70% to 95%, or from 75% to 90% of the height Hsw (extending from the bottom to the perimeter) of the side wall 104 (which may be the same as the height of the pocket H_{P}). The row of elements 117 extending along the side walls 104 extend in the width direction (which extends perpendicular to the height direction) of the side wall 104. The elements 116 may form a single row along the side wall 104, i.e. there is not more than one element 116 provided in the height direction of the side wall 104. The elements may have a width -extending along the width of the side wall- of 50% to 200% of the element's height, or of 70% to 150% of the element's height, or 80% to 140% of the element's height. The elements 116 within a row of elements 117 may all have the same shape. Alternatively, the elements 116 within a row of elements 117 may have different shapes. In such configurations it is, however, preferred, that the elements 116 have substantially the same height H_{E}, i.e. the height may not differ by more than 20% from the element having the smallest height to the element with the largest height.The row of elements 117 may be spaced apart from the perimeter 102 (and from the rim 118) as well as from the bottom 103 of the pocket 100.

The row of elements 117 may form a closed chain of elements 116 along the side wall 104, such that neighboring elements are in contact with each other. Neighboring elements may not overlap each other.

The pocket 100 may fold along the area where the elements within a row of elements 117 (forming a closed chain of elements) are in contact with each other. Thereby, a hinge 121 is provided in the side wall 104 along which the pocket 100 folds when a pressure is applied substantially (or exactly) in the direction parallel to the height of the pocket. "Substantially" in the direction parallel to the height of the pocket means ± 35%.

Elements 116 provided as several elliptical, oval, eye shaped, or circular elements, and having an opening 127 therein, may have an outer perimeter 125 and an inner perimeter 126, the inner perimeter 126 defining the size and shape of the opening 127.

The bottom may, in addition to the first material, comprise a fourth material. The first material may be a film and the fourth material may be a nonwoven web.

In such a pocket, the nonwoven may form the first surface of the bottom and the film may form the second surface of the pocket. The film may have the second attachment material provided on its outwardly facing surface, i.e. on the second surface of the pocket. The nonwoven web may be in contact with the skin of the wearer when the pocket is in use and the pocket is in its collapsed configuration. The film (hence, the first material) may be made of polyethylene or co-polymers thereof. The basis weight of the first material may be from 8 g/m² to 430 g/m ², or from 8 g/m² to 30 g/m², or from 10 g/m² to 25 g/m². The nonwoven web (hence, the fourth material) may be, for example, a carded or spunbond nonwoven web. The nonwoven web may be made of, for example, polyolefin fibers, polypropylene fibers, or polyethylene terephthalate fibers. The basis weight of the nonwoven web forming the fourth material may be at least 8 g/m², or at least 10 g/m², or at least 12 g/m². The basis weight of the nonwoven web forming the fourth material may not be more than 40 g/m², or not more than 30 g/m².

The pocket may further comprise a grip 120 or handle provided at or adjacent the perimeter 102 of the pocket 100. The grip 120 allows for easier disposal of the pocket after use. In a pocket 100 comprising a rim 118 at the perimeter, the grip 120 may be joined to the surface of the rim 118 which is opposite to the rim's first surface 122, and which faces away from the skin of the wearer in use.

In pockets 100 provided with a rim 118, the rim may comprise two hinges 119 which enable folding the rim 118 upward away from the side wall 104 and bottom 103. The hinges 119 may be configured such that the rim 118 can be folded symmetrically into two substantially equal halves. After removal of the pocket 100 from the wearer's skin, at least a portion of the first attachment material may still be present on the first surface 122 of the rim 118 of the pocket 100. Therefore, due to the first attachment material, folding the rim 118 at the hinges 119 facilitates attachment of the two rim halves to each other, thereby closing the pocket 100 after use.

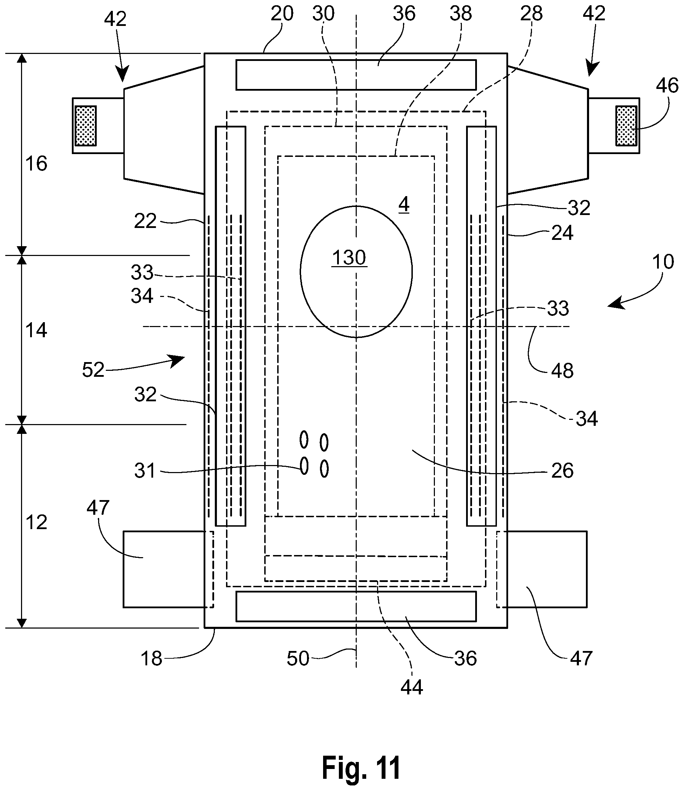

The pocket may be comprised by a kit, the kit comprising one or more pockets and one or more articles, the articles being selected from disposable diapers, disposable absorbent pants, disposable inserts and outer covers. The kit may comprise a higher number of pockets comparted to the number of articles. The kit may be provided in a package. The articles comprised by the kit may have a designated target area (exemplarily shown as 130 in Fig. 11) intended for attachment to the second attachment material. The designated target area may be indicated by one or more indicia, such as printed graphics. The articles may comprise a topsheet and the designated target area may be provided on or with the tophsheet. When the article is an outer cover, the designated target area may be provided on or with the surface of the outer cover that is facing towards the skin of the wearer in use.

The outer cover may be used with only the pocket or may be used with an absorbent insert and the pocket.

An array of kits, wherein the kits of the array differ by the pocket, the article or the pocket and article is also provided herein. The array may be provided adjacent to each other on the shelf in a store.

The disposable defecation pocket of the present invention considerably simplifies cleanup of soiled areas on the wearer's skin, as it can limit fecal material being smeared on the skin to the area of the opening. Fecal material can be effectively isolated from areas of the skin and article outside of the pocket. Thereby, skin health can be improved. Moreover, as the pocket can be deposited independently from the article, the amount of waste can be reduced given that the article can be continued to use, e.g., when the article is a diaper, absorbent pant or absorbent insert and no or little urine has been deposited in the article at the time of defecation.

The pocket can be equally used in combination with disposable articles, such as disposable diapers, absorbent pants and inserts, and with cloth diapers and regular underpants.

### Diaper and absorbent pant as article to be used in conjunction with the pocket

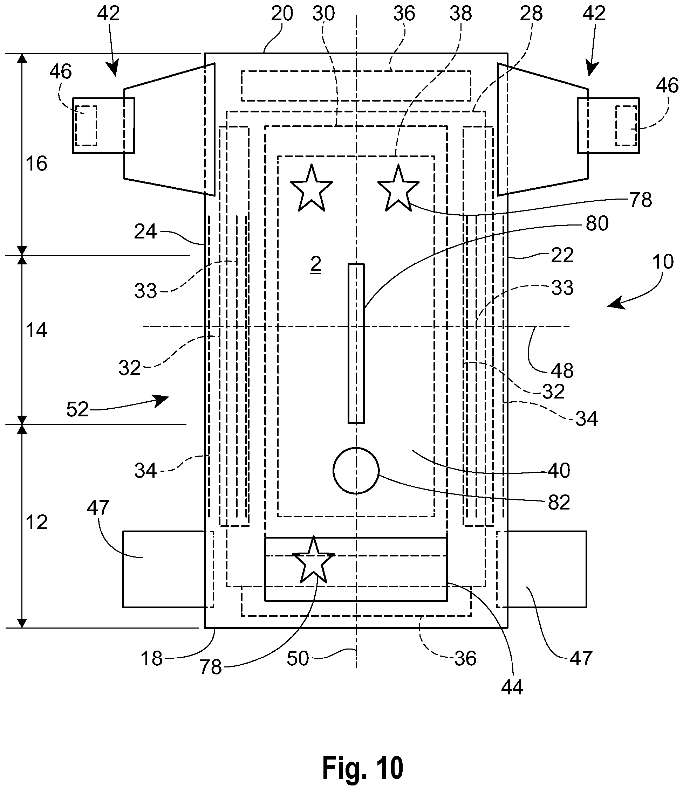

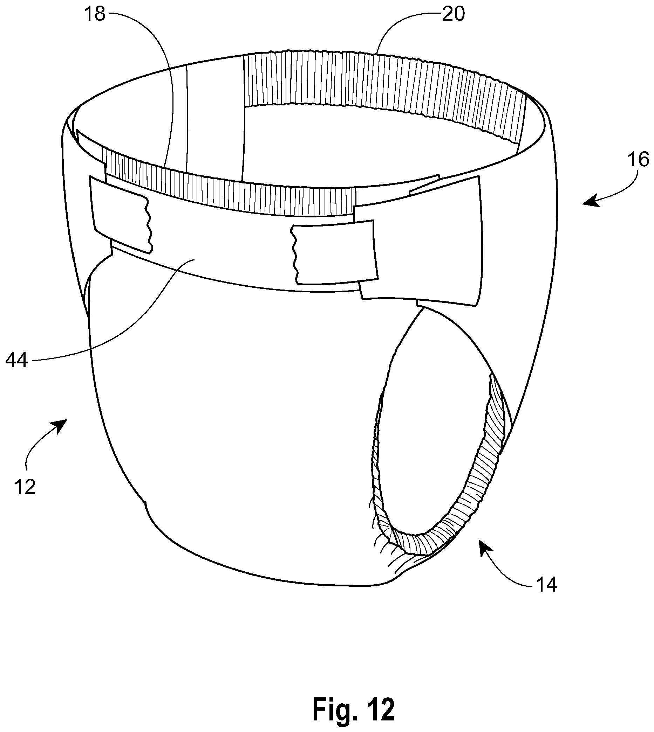

An example article 10 according to the present disclosure, shown in the form of a taped diaper (herein also referred to as 'diaper'), is represented in Figs. 10-12. Fig. 10 is a plan view of the example article 10, garment-facing surface 2 facing the viewer in a flat, laid-out state (i.e., no elastic contraction). Fig. 11 is a plan view of the example article 10 of Fig. 10, wearer-facing surface 4 facing the viewer in a flat, laid-out state. Fig. 12 is a front perspective view of the article 10 of Figs. 10 and 11 in a fastened configuration. The article 10 of Figs. 10-12 is shown for illustration purposes only as the present disclosure may be used for making a wide variety of diapers, including adult incontinence products, absorbent pants, and absorbent inserts, for example.

The article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the article 10. The article 10 may comprise a front end edge 18, a back end edge 20 opposite to the front end edge 18, and longitudinally extending, transversely opposed side edges 22 and 24 defined by the chassis 52.

The article 10 may comprise a liquid permeable topsheet 26, a liquid impermeable backsheet 28, and an absorbent core 30 positioned at least partially intermediate the topsheet 26 and the backsheet 28. The article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material, may cover a garment-facing side of the backsheet 28. The article 10 may comprise back ears 42 in the back waist region 16. The back ears 42 may comprise fasteners 46 and may extend from the back waist region 16 of the article 10 and attach (using the fasteners 46) to the landing zone area or landing zone material 44 on a garment-facing portion of the front waist region 12 of the article 10. The article 10 may also have front ears 47 in the front waist region 12. The article 10 may have a central lateral (or transverse) axis 48 and a central longitudinal axis 50. The central lateral axis 48 extends perpendicular to the central longitudinal axis 50.

In other instances, the article may be in the form of an absorbent pant having permanent or refastenable side seams. Suitable refastenable seams are disclosed in U.S. Pat. Appl. Pub. No. 2014/0005020 and U.S. Pat. No. 9,421,137.

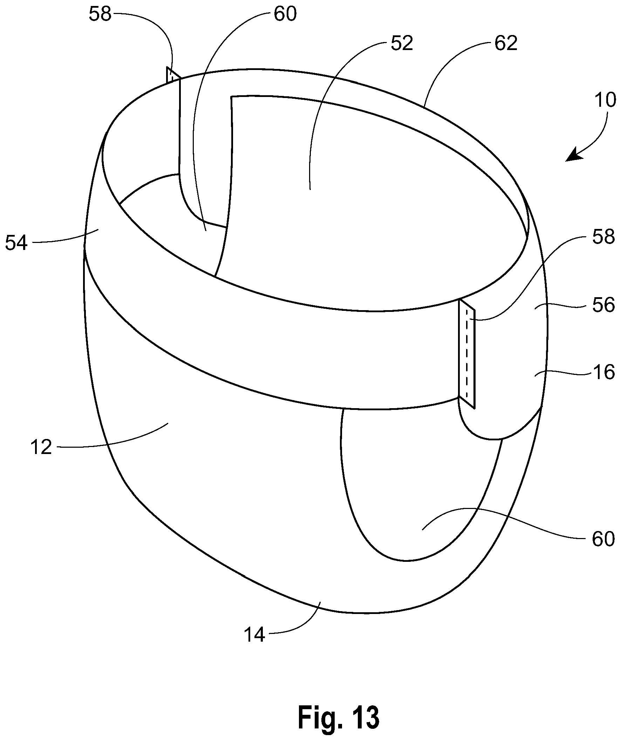

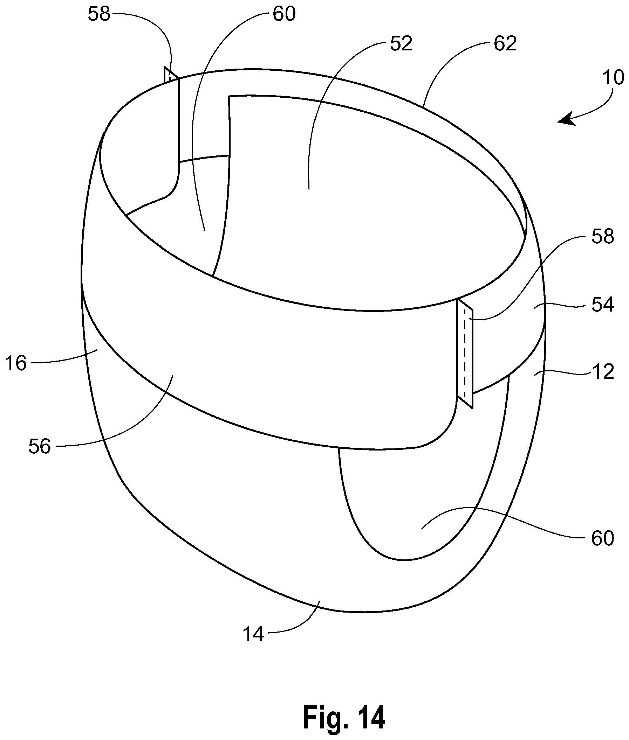

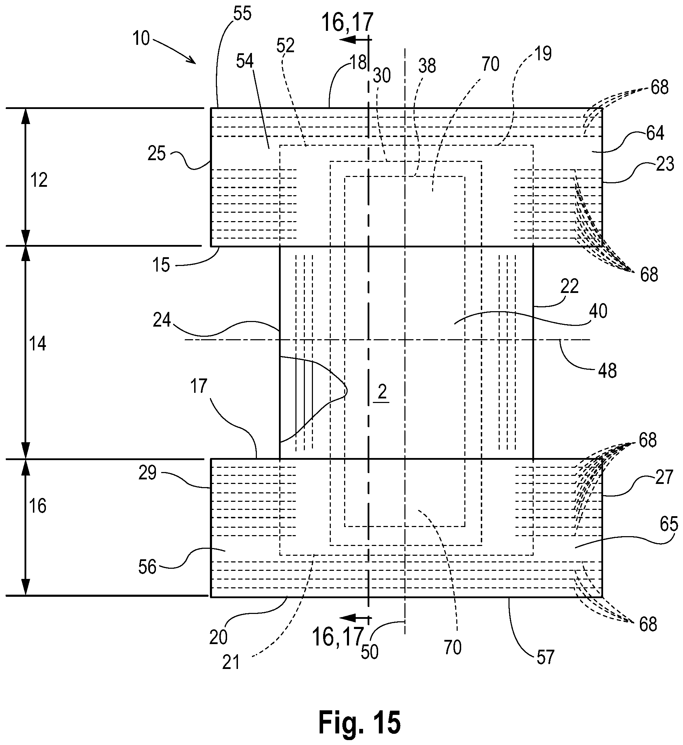

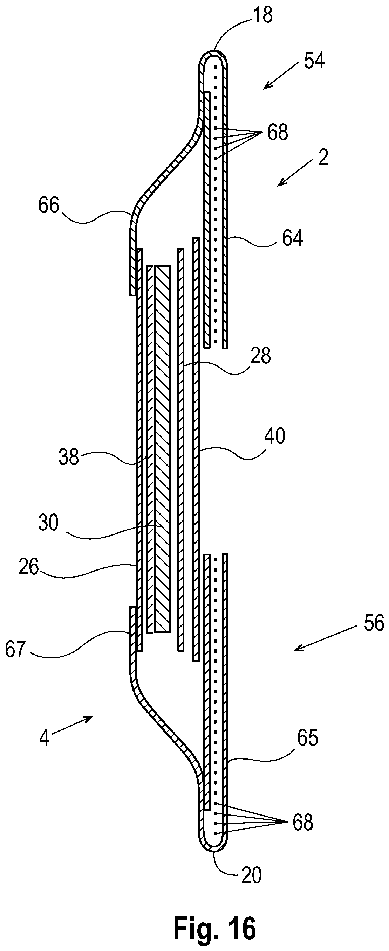

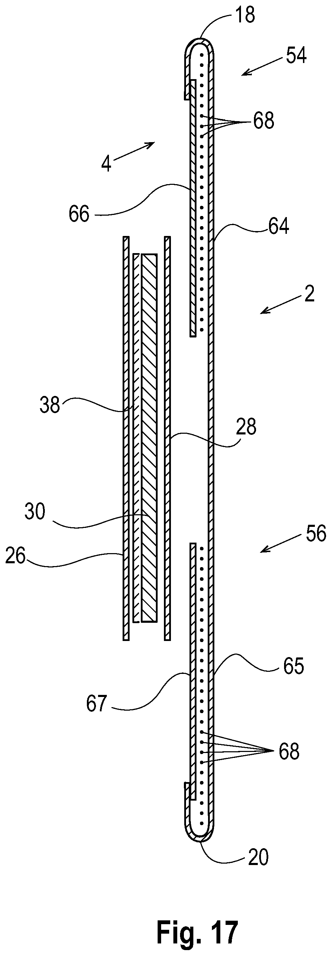

Referring to Figs. 13-17, an example article 10 in the form of an absorbent pant is illustrated. Fig. 13 is a front perspective view of the article 10. Fig. 14 is a rear perspective view of the article 10. Fig. 15 is a plan view of the article 10, laid flat, with the garment-facing surface facing the viewer. Elements of Fig. 13-17 having the same reference number as described above with respect to Figs. 10-12 may be the same element (e.g., absorbent core 30). Fig. 16 is an example cross-sectional view of the article taken about line 16-16 of Fig. 15. Fig. 17 is an example cross-sectional view of the article taken about line 17-17 of Fig. 15. Figs. 16 and 17 illustrate example forms of front and back belts 54, 56. The article 10 may have a front waist region 12, a crotch region 14, and a back waist region 16. Each of the regions 12, 14, and 16 may be 1/3 of the length of the article 10. The article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 10-12. The article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the article 10 in the form of an absorbent pant has two leg openings 60 and a waist opening circumference 62. The side seams 58 may be permanently joined using adhesives or bonds, for example, or may be refastenably closed using hook and loop fasteners, for example.

### Top sheet

The topsheet 26 is the part of the article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be water permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 11, element 31), may have any suitable three-dimensional features, and/or may have a plurality of embossments (e.g., a bond pattern). The topsheet may comprise a variable basis weight nonwoven material, such as those described in U.S. Pat. Appl. No. 2017/0191198. The topsheet may be apertured by overbonding a material and then rupturing the overbonds through ring rolling, such as disclosed in U.S. Patent No. 5,628,097, to Benson et al., issued on May 13, 1997, and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The topsheet may comprise a designated target area to indicate the position to which the second attachment material of the pocket is intended to be attached.

### Backsheet

The backsheet 28 is generally that portion of the article 10 positioned proximate to the garment-facing surface of the absorbent core 30. The backsheet 28 may be joined to portions of the topsheet 26, the outer cover material 40, the absorbent core 30, and/or any other layers of the article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials, such as films, which permit vapors to escape from the article, while still preventing, or at least inhibiting, bodily exudates from passing through the backsheet. The backsheet 28 may be coterminous with the outer cover material 40.

### Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material or a variable basis weight nonwoven material.

### Absorbent Core

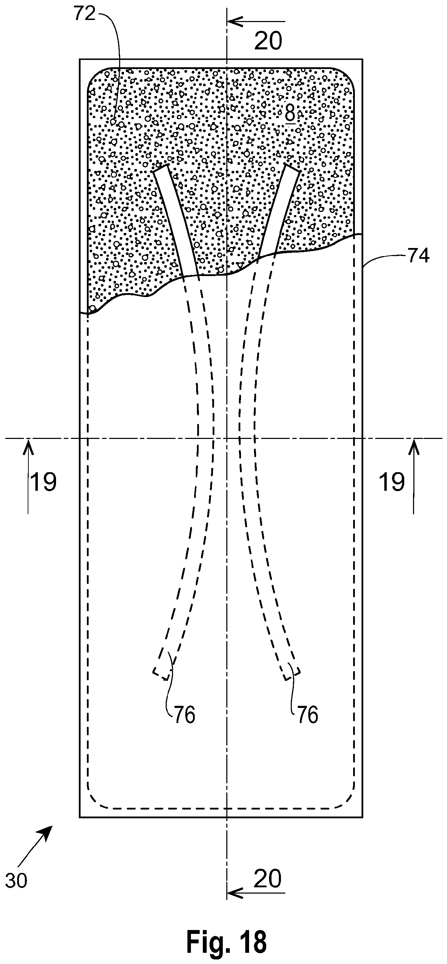

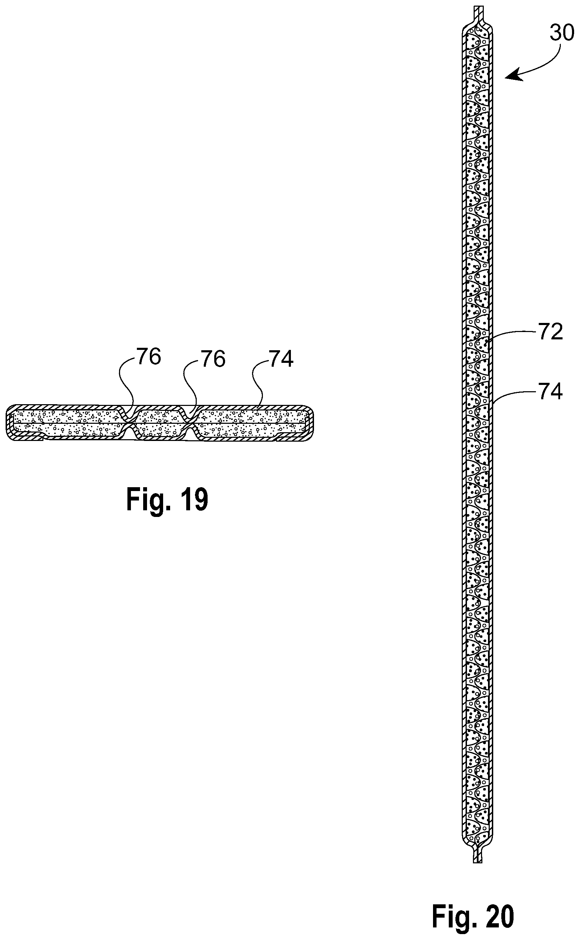

As used herein, the term "absorbent core" 30 refers to a component of the article 10 disposed in the article for absorbing and containing liquid such as urine received by the article. The absorbent core thus typically has a high absorbent capacity. An example absorbent core 30 is schematically shown in Figs. 18-20. The absorbent core comprises an absorbent material 72, that is typically enclosed within or sandwiched between a core bag 74.

The core wrap may be a single material that is folded and attached to itself, or it may comprise a separate top layer and bottom layer that may be bonded, adhesively joined, or otherwise joined together. The top and bottom layers of the core wrap may be the same or different. The absorbent material typically comprises superabsorbent particles which are optionally mixed with cellulose fibers. As used herein, "absorbent core" does not include any acquisition-distribution systems, top sheet, or backsheet of the article.

The example absorbent core 30 shown in isolation in Figs. 18-20 is in the dry state (before use). The absorbent core may typically have a generally rectangular shape as defined by its longitudinal edges and transversal front edge and back edge or may have other shapes.

Absorbent material 72 may be deposited as an absorbent layer having a generally rectangular outline, as represented in Fig. 18. A wide variety of absorbent cores may also be used. The absorbent material 72 layer may also have a non-rectangular perimeter ("shaped" core), in particular, the absorbent material 72 may define a tapering along its width towards the central region of the core (or "dog-bone" shape). In this way, the absorbent material deposition area may have a relatively narrow width in an area of the core intended to be placed in the crotch region of the article. This may provide for example better wearing comfort. Other shapes can also be used such as a "T" or "Y" or "hourglass" for the area of the absorbent material.

The absorbent material 72 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists, or consists essentially, of SAP. The absorbent material may also be a high internal phase emulsion foam

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from about 20 g/g to about 40 g/g. "Superabsorbent polymer particles", as used herein, refers to a superabsorbent polymer material which is in particulate form so as to be flowable in the dry state.

### Barrier Leg Cuffs/Leg Elastics

Referring to Figs. 10 and 11, for example, the article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the article 10 so it can extend upwards from a wearer-facing surface 4 of the article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the article 10 proximate to the chassis side edges 22, 24 to help form a seal around the legs of the wearer. The leg elastics 34 may extend at least within the crotch region 14.

### Elastic Waistband

Referring to Figs. 10 and 11, the article 10 may comprise one or more elastic waistbands 36. The elastic waistbands 36 may be positioned on the garment-facing surface 2 or the wearer-facing surface 4. Alternatively, the elastic waistbands 35 may be positioned intermediate the topsheet and the backsheet. As an example, a first elastic waistband 36 may be present in the front waist region 12 near the front belt end edge 18 and a second elastic waistband 36 may be present in the back waist region 16 near the back end edge 20. The elastic waistbands 36 may aid in sealing the article 10 around a waist of a wearer and at least inhibiting bodily exudates from escaping the article 10 through the waist opening circumference. In some instances, an elastic waistband may fully surround the waist opening circumference of an article.

### Acquisition Materials

Referring to Figs. 10, 11, 16, and 17, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, formed films, apertured formed films, cellulosic materials, cross-linked cellulosic materials, air laid cellulosic nonwoven materials, spunlace materials, or combinations thereof, for example. The acquisition material may be rectangular or may be shaped, such as hourglass shaped. Typically, an acquisition material 38 may have a width and length that are smaller than the width and length of the topsheet 26. The acquisition material may be a secondary topsheet in the feminine pad context. The acquisition materials may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

### Landing Zone

Referring to Figs. 10 and 11, the article 10 may have a landing zone area 44 that is formed in a portion of the garment-facing surface 2 of the outer cover material 40. The landing zone area 44 may be in the back waist region 16 if the article 10 fastens from front to back or may be in the front waist region 12 if the article 10 fastens back to front. In some instances, the landing zone 44 may be or may comprise one or more discrete nonwoven materials that are attached to a portion of the outer cover material 40 in the front waist region 12 or the back waist region 16 depending upon whether the article fastens in the front or the back. In essence, the landing zone 44 is configured to receive the fasteners 46 and may comprise, for example, a plurality of loops configured to be engaged with, a plurality of hooks on the fasteners 46, or vice versa.

### Wetness Indicator/Graphics

Referring to Fig. 10, the articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

### Front and Back Ears

Referring to Figs. 10 and 11, as referenced above, the article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. Any ears with fasteners may be configured to engage an opposing ear or the landing zone or landing zone area 44. One set of ears may comprise primary fasteners and the other set of ears may comprise secondary fasteners. The ears, or portions thereof, may be elastic or may have elastic panels. The ears may be shaped. The ears may be integral (e.g., extension of the outer cover material 40, the backsheet 28, and/or the topsheet 26) or may be discrete components attached to a chassis 52 of the article on a wearer-facing surface 4, on the garment-facing surface 2, or intermediate the two surfaces 4, 2. The back ears may comprise an elastic film sandwiched between two nonwoven materials forming a laminate.

### Packages

The articles of the present disclosure may be placed into packages. The packages may comprise polymeric films, paper, and/or other materials. Graphics and/or indicia relating to properties of the articles may be formed on, printed on, positioned on, and/or placed on outer portions of the packages. Each package may comprise a plurality of articles. The articles may be packed under compression so as to reduce the size of the packages, while still providing an adequate number of articles per package.

As is set out above, a package can also comprise a kit with one or more than one pocket and one or more than one article.

### Arrays

"Array" means a display of packages comprising disposable absorbent articles of different article constructions (e.g., different elastomeric materials [compositionally and/or structurally] in the side panels, back ears, side flaps and/or belts flaps, different graphic elements, different product structures, fasteners, waistbands, or lack thereof). The array may also mean a display of packages comprising kits of one or more pocket and one or more article. The kits within the array differ from each other in differences in the articles, the pockets or both. The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

### Test method

### Low Surface Tension Fluid Strikethrough Time Test

The low surface tension fluid strikethrough time test is used to determine the amount of time it takes a specified quantity of a low surface tension fluid, discharged at a prescribed rate, to fully penetrate a sample of a web (and other comparable barrier materials) which is placed on a reference absorbent pad.

For this test, the reference absorbent pad is 5 plies of Ahlstrom grade 989 filter paper (10 cm×10 cm) and the test fluid is a 32 mN/m low surface tension fluid.

This test is designed to characterize the low surface tension fluid strikethrough performance (in seconds) of webs and components (such as the side wall and the bottom of the pocket of the present invention) intended to provide a barrier to low surface tension fluids, such as runny BM, for example.

Lister Strikethrough Tester: The instrumentation is like described in EDANA ERT 153.0-02 section 6 with the following exception: the strike-through plate has a star-shaped orifice of 3 slots angled at 60 degrees with the narrow slots having a 10.0 mm length and a 1.2 mm slot width. This equipment is available from Lenzing Instruments (Austria) and from W. Fritz Metzger Corp (USA). The unit needs to be set up such that it does not time out after 100 seconds.

Reference Absorbent Pad: Ahlstrom Grade 989 filter paper, in 10 cm×10 cm areas, is used. The average strikethrough time is 3.3+0.5 seconds for 5 plies of filter paper using the 32 mN/m test fluid and without the web sample. The filter paper may be purchased from Empirical Manufacturing Company, Inc. (EMC) 7616 Reinhold Drive Cincinnati, Ohio 45237.

Test Fluid: The 32 mN/m surface tension fluid is prepared with distilled water and 0.42+/-0.001 g/liter Triton-X 100. All fluids are kept at ambient conditions.

Electrode-Rinsing Liquid: 0.9% sodium chloride (CAS 7647-14-5) aqueous solution (9 g NaCl per 1L of distilled water) is used.

### Test Procedure

- All testing is performed in a room maintained at about 23°C ± 2°C and about 50% ± 2% relative humidity. The Ahlstrom filter paper and test articles are conditioned in this controlled environment for 24 hours and 2 hours before testing.
- Ensure that the surface tension is 32 mN/m +/-1 mN/m. Otherwise remake the test fluid.
- Prepare the 0.9% NaCl aqueous electrode rinsing liquid.
- Ensure that the strikethrough target (3.3+/-0.5 seconds) for the Reference Absorbent Pad is met by testing 5 plies with the 32 mN/m test fluid as follows:
- Neatly stack 5 plies of the Reference Absorbent Pad onto the base plate of the strikethrough tester.
- Place the strikethrough plate over the 5 plies and ensure that the center of the plate is over the center of the paper. Center this assembly under the dispensing funnel.
- Ensure that the upper assembly of the strikethrough tester is lowered to the pre-set stop point.
- Ensure that the electrodes are connected to the timer.
- Turn the strikethrough tester "on" and zero the timer.
- Using the 5 mL fixed volume pipette and tip, dispense 5 mL of the 32 mN/m test fluid into the funnel.
- Open the magnetic valve of the funnel (by depressing a button on the unit, for example) to discharge the 5 mL of test fluid. The initial flow of the fluid will complete the electrical circuit and start the timer. The timer will stop when the fluid has penetrated into the Reference Absorbent Pad and fallen below the level of the electrodes in the strikethrough plate.
- Record the time indicated on the electronic timer.
- Remove the test assembly and discard the used Reference Absorbent Pad. Rinse the electrodes with the 0.9% NaCl aqueous solution to "prime" them for the next test. Dry the depression above the electrodes and the back of the strikethrough plate, as well as wipe off the dispenser exit orifice and the bottom plate or table surface upon which the filter paper is laid.
- Repeat this test procedure for a minimum of 3 replicates to ensure the strikethrough target of the Reference Absorbent Pad is met. If the target is not met, the Reference Absorbent Pad may be out of spec and should not be used.
- After the Reference Absorbent Pad performance has been verified, nonwoven web samples may be tested.
- Precondition the test components or materials at about 23° C.±2° C. And about 50%±2% relative humidity for two hours prior to testing. To obtain a specimen, using scissors cut a test specimen 70 mm long from the bottom component of the pocket. Proceed likewise for the the test specimen of the side wall component of the pocket.
- Place the specimen centered over the port of the strike through plate. The specimen should completely cover the port with the first surface of the side wall and bottom, respectively, directed toward the port. Gently extend the specimen taut in its longitudinal direction so that the specimen lies flat against the upper test plate. Adhesive tape is applied to secure the specimen to the test plate in its extended state for testing. Tape should not cover any portion of the measurement port.
- Ensure that the upper assembly of the strikethrough tester is lowered to the pre-set stop point.
- Ensure that the electrodes are connected to the timer. Turn the strikethrough tester "on" and zero the timer.
- Run as described above.
- Repeat this procedure for three samples obtained from three pockets. Average the six values and report as the 32 mN/m low surface tension strikethrough time to the nearest 0.1 seconds.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A disposable defecation pocket (100) for containing feces, the pocket (100) having an opening (101), a perimeter (102) around the opening (101), a bottom (103), a side wall (104) extending from the perimeter (102) to the bottom (103), the side wall (104) and the bottom (103) each having a first surface (105, 106) facing towards the inside of the pocket (100) and a second surface (107, 108) facing outwardly;
The pocket (100) being able to expand from a flat, collapsed configuration to an erected configuration, wherein the erected configuration provides a void volume (109) inside the pocket (100), the pocket (100) being, at least partly, in its erected configuration in the absence of any applied pressure;
the pocket (100) comprising:
- a first material (110) forming the bottom (103),
- a second material (111) forming the side wall (104),
wherein the first and second material (110, 111) is the same or different;
- a first attachment material continuously provided along the perimeter (102), the first attachment material enabling attachment of the perimeter 102) to the skin around the anus of a wearer;
- a second attachment material provided on the second surface (108) of the bottom (103), the second attachment material enabling attachment of the bottom (103) to a surface of an article selected from the group consisting of a diaper, an absorbent pant, an absorbent insert, an outer cover, and underpants.

2. The defecation pocket (100) of claim 1, wherein the pocket (100) converts from its flat compressed configuration into its least partly or completely erected configuration on its own motion in the absence of any applied pressure.

3. The defecation pocket (100) of claim 1 or 2, wherein the first attachment material is a body adhesive.

4. The defecation pocket (100) of any of the preceding claims, wherein the second attachment material is an adhesive or a mechanical fastener.

5. The pocket (100) of any of the preceding claims, wherein the size and shape of the perimeter enables attachment around the anus of a wearer, including the area between the anus and the genitals of a wearer.

6. The pocket of any of the preceding claims, wherein the perimeter (102) is formed by a rim (118), the rim (118) comprising an outer perimeter (124) and an inner perimeter (123), the inner perimeter (123) forming the opening (101), the rim (118) comprising a first surface (122) intended to be in contact with the skin of the wearer when the pocket (100) is in use, wherein the first attachment material of the perimeter (102) is provided on the first surface (122) of the rim (118).

7. The pocket (100) of any of the preceding claims, wherein the pocket (100) has a minimum height in its flat, collapsed configuration and a maximum height in its erected configuration, and wherein the pocket erects to at least 30%, or at least 50% of its maximum height in the absence of any applied forces.

8. The pocket (100) of any of the preceding claims, wherein the side wall (104) comprises a hinge (121), the hinge (121) providing a fold line along which the pocket (100) folds when being compressed from its erected configuration to its flat, collapsed configuration.

9. The pocket (100) of any of the preceding claims, wherein the side wall (104) comprises a third material (112), the third material (112) being provided discontinuously and being attached to the second material (111), the combination of the second and third material (111, 112) enabling at least partial erection of the pocket in the absence of external forces applied on the pocket.

10. The pocket (100) of claim 9, wherein the third material (112) is a foam, such as a expanded polyethylene foam.

11. The pocket (100) of claim 9 or 10, wherein the third material (112) is provided as several, eye-shaped, oval, elliptical or circular elements (116), each element having an opening (127) therein, and wherein the elements (116) are applied as a row (117) extending along the side wall (104) in a direction perpendicular to the height (H_{P}) of the pocket (100) and perpendicular to the height (Hsw) of the side wall (104).

12. The pocket (100) of claim 11, wherein the elements (116) are eye-shaped.

13. The pocket (100) of claim 11 or 12, wherein the row of elements (117) is spaced from the perimeter (102) and spaced from the bottom (103) of the pocket (100).

14. The pocket (100) of any of claims 11 to 13, wherein the row of elements (117) forms a chain of elements along the side wall (104) and wherein neighboring elements are in contact with each other but do not overlap with each other.

15. The pocket (100) of any of the preceding claims, wherein the bottom (103) has a low surface tension fluid strikethrough time of at least 100 seconds and the side wall (104) has a low surface tension fluid strikethrough time of at least 50 seconds, as measured according to the test method set out herein.

16. The pocket of any of the preceding claims further comprising a grip (120) at or adjacent to the perimeter (102).

17. A kit comprising one or more pockets (100) of any of the preceding claims and one or more articles, each article having a designated target area to which the second attachment material is intended to be attached.
